# EUROPEAN PATENT APPLICATION

(11) **EP 3 087 992 A1**
(43) Date of publication of application: **02.11.2016**
(21) Application number: 15165684.0
(22) Date of filing: 29.04.2015
(51) Int. Cl.: A61K 36/87, A61K 36/064

(54) **COMPOSITIONS COMPRISING YEAST CELLS AND POLYPHENOLS AND THEIR USE**

(71) Applicant: Institut National d'Etudes Superieures Agronomiques de Montpellier (Montpellier SupAgro), 34060 Montpellier Cedex 1 (FR); Institut National de la Recherche Agronomique (INRA), 75338 Paris Cedex 07 (FR); Danstar Ferment AG, 6300 Zug (CH)
(72) Inventor: Vernhet, Aude, 34000 Montpellier (FR); Mekoue Nguela, Julie, 34070 Montpellier (FR); Brillouet, Jean-Marc, 34000 Montpellier (FR); Sieczkowski, Nathalie, 31702 Blagnac (FR)
(74) Representative: Cabinet Armengaud Aîné

(57) **Abstract**

The present disclosure concerns a composition comprising yeast cells and at least one polyphenols having a molecular weight above 620 Da, the polyphenols are adsorbed and retained in the cytoplasm of the yeast cell. The present disclosure also concerns using the composition in cosmetic, dermatological, nutritional and / or pharmaceutical fields, as well as in winemaking to protect the wine against oxidation, therefore preserving the wine quality, color and aromas, and as an alternative to ageing of wine on lees.

## Description

### TECHNOLOGICAL FIELD

The present disclosure relates to compositions comprising yeast cells and at least one polyphenol and their uses.

### BACKGROUND

Polyphenols, which are greatly represented in edible plants, have recently attracted great interest due to their antioxidant properties. The evidence for chemopreventive, anti-inflammatory, and cardioprotective roles of these polyphenols from teas, wines, and fruits is rapidly growing. Polyphenols may do even more than prevent disease; they may be used in cosmetics making individuals more youthful looking.

Polyphenols can also be used to protect the color and phenolic structure of wines. It is known to winemakers that addition of polyphenols during aging process of wine can help prevent excessive oxidation that can result in loss of structure and freshness. However, beyond a certain amount, the polyphenols start having a negative sensory impact on the wine and can even denature the wine.

In the prior art, the envelopment of small molecules in solid particles, liquid droplets or gases in a yeast is growing applications in pharmaceutical, cosmetic and food. The eukaryotic structure of yeast cell made it a potential excellent encapsulating wall material and its natural properties made it many benefits over other microencapsulation techniques. In Shi et al., 2007 (International Journal of Pharmaceutics, 349, 2008, p83-93), they reported the encapsulation of resveratrol into modified yeast (yeast cells pre-treated with a plasmolyser to remove most of the cytoplasmic materials that occupies the cells).

Interactions between yeast cells and polyphenols, have also been studied during fermentation or ageing.²⁻⁵ It has been shown that yeast cells influence the phenolic composition of wines, particularly due to their ability to adsorb some of them. Up to now, this adsorption has mainly been attributed to cell walls, though it has been supposed that low molecular weight polyphenols (monomers and dimers) could enter the periplasmic space through the wall pores and interact with the plasma membrane.⁶

According to Zlontnik et al (J. Bacteriol, 159, 1984, p. 1018-1026), the structure of the yeast cell wall is permeable to both small polar and apolar molecules in aqueous solutions, but the size (or molecular weight) and polarity properties of the molecules are the two limiting factors. Scherrer et al (J. Bacteriol, 118, 1974, p. 534-540) reported that a molecule, with a molecular radius smaller than 0.81 nm or molecular weight lower than 620 Da, could penetrate through the yeast cell wall freely.

### BRIEF SUMMARY

In accordance with the present invention there is provided a composition comprising yeast cells and at least one polyphenols having a molecular weight above 620 Da, the polyphenols are adsorbed and retained in the cytoplasm of the yeast cell.

In accordance with the present invention there is provided a use of the composition described herein in cosmetic, dermatological, nutritional and/or pharmaceutical fields.

In accordance with the present invention there is provided a use of the composition in winemaking to protect the wine against oxidation, therefore preserving the wine quality, color and aromas. In embodiments, the composition can be used as an alternative to ageing on lees.

### BRIEF DESCRIPTION OF THE DRAWINGS

Having thus generally described the nature of the invention, reference will now be made to the accompanying drawings, showing by way of illustration, a preferred embodiment thereof, and in which:
**Figure 1** shows untreated (A, C) and treated (B,D) yeast cells with grape skin proanthocyanidins under light and epifluorescence microscopy;
**Figure 2** shows confocal images of untreated and treated yeast cells (Y, IY, A-IY) and untreated and treated yeast yell walls (CW) with grape skin proanthocyanidins;
asterisk focuses on fluorescent clotted cytoplasm; white arrows: clotted cell walls;
blue arrow: remnants of cytoplasm out of the cells. Y means whole yeast cells, IY means Inactivated Yeast cells and A-IY means yeast inactivated after autolysis.
**Figure 3** shows (A) Confocal image of treated cells with grape skin proanthocyanidins and (B) spectral analysis showing the auto-fluorescence emission spectrum of grape tannins (λem 430-800 nm range)¹⁴. ROI: region of interest.
**Figure 4** shows TEM images at diverse magnifications of (A-C) untreated and (D-F) treated yeast cells (Y) and cell wall (CW) with grape skin proanthocyanidins; red arrows: mannoproteins; blue arrows: cell wall thickness.
**Figure 5** shows TEM images at diverse magnifications of (A-C) untreated and (D-F) treated yeast cells (Y) and cell wall (CW) with wine polyphenol pool; red arrows: mannoproteins; blue arrows: antennary oligo(manno)-saccharides.
**Figure 6** shows TEM images of (A) treated yeast cell wall with wine polyphenol pool, showing clotted outer and inner mannoproteins, (B) a treated cell showing clotting of the plasmalemma, and (C) drawing of plasmolysed outer region of untreated and treated yeast cells. cw: polysaccharidic cell wall; mp: mannoproteins; pe: periplam; pl: plasmalemma; red circles: GPI anchor; blue arrows: contrasted knots in the inner layer of mannoproteins; green arrows: contrasted knots at the outer layer of mannoproteins ; red arrows: clumps of clotted plasmalemma proteins ; on scale.

### DETAILED DESCRIPTION

The present description is based on the unexpected discovery that polyphenols with a molecular weight above 620 Da could cross the yeast cell envelope barrier (cell wall and plasma membrane) of yeasts and interact with their cytoplasm constituents.

Therefore, the present disclosure provides a composition comprising yeast cells and at least one polyphenols having a molecular weight above 620 Da, the polyphenols are adsorbed and retained in the cytoplasm of the yeast cell.

Polyphenols are a class of chemical compounds comprising a hydroxyl group (-OH) bounded directly to an aromatic hydrocarbon group. This large group of compounds can be broadly separated into two categories: flavonoids and non-flavonoids. Exemplary Flavonoids include, but are not limited to flavonols, flavanols, flavanonols anthocyanidins and anthocyanins. Exemplary non-flavonoids include, but are not limited to phenolic acids, stilbenoids and hydrolysable tannins. Exemplary hydrolysable tannins include, but are not limited to ellagitannins and gallotannins. The ellagitannins are formed primarily from the oxidative linkage of galloyl groups in 1,2,3,4,6-pentagalloyl glucose. The gallotannins are polymers formed when gallic acid, a polyphenol monomer, esterifies and binds with the hydroxyl group of a polyol carbohydrate such as glucose.

In embodiments, the at least one polyphenols can be flavonols, flavanonols, flavanols, anthocyanidins, anthocyanins, phenolic acids, stilbenoids, hydrolysable tannins or mixtures thereof. In still embodiments, the at least one polyphenols are selected from the group consisting of flavonols, flavanonols, flavanols, anthocyanidins, anthocyanins, phenolic acids, stilbenoids, hydrolysable tannins and mixtures thereof. In embodiments, the at least one polyphenols can be hydrolysable tannins. In further embodiments, the at least one polyphenols can be ellagitannins or gallotannins. In still embodiments, the at least one polyphenols are selected from the group consisting of ellagitannins and gallotannins. In embodiments, the at least one polyphenols can be flavan-3-ols. In still some embodiments, the at least one polyphenols can be formed by polymerization of flavan-3-ols. The at least one polyphenols formed by polymerization of flavan-3-ols are also called condensed tannins or proanthocyanidins. In embodiments, the at least one polyphenols can be proanthocyanidins.

Polyphenols are found in numerous fruits and vegetable. Hydrolysable tannins and proanthocyanidins are abundant in various plants, especially in grape skins and seeds, lingonberries and bilberries, cranberries, gallnuts, sumac, witch hazel, tea leaves and some types of wood, such as quebracho wood. Proanthocyanidins are also found in wine. Proanthocyanidins have a high level of antioxidant activity, and various studies have demonstrated their efficacy in reducing the blood pressure, reducing platelet aggregation and counteracting the progress or onset of disorders of the cardiovascular system. Proanthocyanidins also possess antibacterial, antiviral, anti-angiogenetic, antitumoral and chemopreventive properties.

In embodiments, the at least one polyphenols can have a molecular weight above about 870 Da. In other embodiments, the at least one polyphenols can have a molecular weight ranging between about 620 Da to about 20 kDa. In further embodiments, the at least one polyphenols can have a molecular weight ranging between about 870 Da and about 14.5 kDa.

In embodiments, the at least one polyphenols having a molecular weight above 620 Da can have a mean degree of polymerization (DP) ranging between about 3 and about 50. In other embodiments, the mean degree of polymerization (DP) of the at least one polyphenols ranges between about 5 and about 35. In still an embodiment, the mean degree of polymerization (DP) of the at least one polyphenols ranges between about 7 and about 25.

In embodiments, the at least one polyphenols having a molecular weight above 620 Da can have a mean degree of polymerization (DP) ranging between about 3 and about 50. In further embodiments, the at least one polyphenols having a molecular weight above 620 Da can have a mean degree of polymerization (DP) ranging between about 5 and about 35. In other embodiments, the at least one polyphenols having a molecular weight above 620 Da can have a mean degree of polymerization (DP) ranging between about 7 and about 25.

In embodiments, the at least one polyphenols having a molecular weight above 870 Da can have a mean degree of polymerization (DP) ranging between about 3 and about 50. In further embodiments, the at least one polyphenols having a molecular weight above 870 Da can have a mean degree of polymerization (DP) ranging between about 5 and about 35. In other embodiments, the at least one polyphenols having a molecular weight above 870 Da can have a mean degree of polymerization (DP) ranging between about 7 and about 25.

In still embodiments, the at least one polyphenols having a molecular weight ranging between about 620 Da to about 20 kDa can have a mean degree of polymerization (DP) ranging between about 3 and about 50. In further embodiments, the at least one polyphenols having a molecular weight ranging between about 620 Da to about 20 kDa can have a mean degree of polymerization (DP) ranging between about 5 and about 35. In other embodiments, the at least one polyphenols having a molecular weight ranging between about 620 Da to about 20 kDa can have a mean degree of polymerization (DP) ranging between about 7 and about 25.

In embodiments, the at least one polyphenols having a molecular weight ranging between about 870 Da and about 14.5 kDa can have a mean degree of polymerization (DP) ranging between about 3 and about 50. In further embodiments, the at least one polyphenols having a molecular weight ranging between about 870 Da and about 14.5 kDa can have a mean degree of polymerization (DP) ranging between about 5 and about 35. In other embodiments, the at least one polyphenols having a molecular weight ranging between about 870 Da and about 14.5 kDa can have a mean degree of polymerization (DP) ranging between about 7 and about 25.

In embodiments, the at least one polyphenols can be isolated and/or extracted from, but not limited to grape skins and/or seeds, lingonberries and bilberries, cranberries gallnuts, sumac, witch hazel, tea leaves and some types of wood, such as quebracho wood. The at least one polyphenols can also be isolated and/or extracted from wine. In embodiments, the at least one polyphenols can be isolated and/or extracted from wine, cranberries or grape skin and/or seeds. In embodiments, the at least one polyphenols can be isolated and/or extracted from wine. In further embodiments, the at least one polyphenols can be isolated and/or extracted from cranberries. In yet embodiments, the at least one polyphenols can be isolated and/or extracted from grape skin and/or seeds.

In all of the embodiments of the present description, the yeast cells can be live yeast cells, dead yeast cells or inactivated yeast.

Inactivated yeast can be obtained by applying lethal conditions to freshly grown yeast in a way to get a totally dead population of yeast. Those lethal conditions includes amongst other things pH or temperature shock, inducing the instant death of all yeast cells, and inducing the partial denaturation and degradation of yeast components (nucleotides, proteins and peptides, cell membranes). Alternatively, inactivated yeast can be obtained by autolysis.

Contrary to inactivated yeast, dead yeast is not obtained through an active step of inactivation of freshly grown yeast; death may be due, for example, to the end of its natural growth during the propagation under anaerobic or aerobic conditions. Exemplary yeasts includes, but are not limited to *Saccharomyces* sp. (for example, from the genus *Saccharomyces arboricolus, Saccharomyces eubayanus, Saccharomyces bayanus, Saccharomyces beticus, Saccharomyces cerevisiae, Saccharomyces fermentati, Saccharomyces kudriadzevii, Saccharomyces mikatae, Saccharomyces paradoxus, Saccharomyces pastorianus* and *Saccharomyces uvarum.), Brettanomyces* sp. (Teleomorph *Dekkera* sp.), *Candida* (Teleomorphs for different species from several genera including *Pichia* sp., *Metschnikowia* sp., *Issatchenkia* sp., *Torulaspora* sp. and *Kluyveromyces* sp.), *Kloeckera* sp. (Teleomorph *Hanseniaspora* sp.), *Saccharomycodes* sp., *Schizosaccharomyces* sp. *Yarrowia sp.* (*Yarrowia lipolytica*) and *Zygosaccharomyces* sp.

In embodiments, the yeast cells can be from *Saccharomyces* sp., *Brettanomyces* sp., *Candida, Kloeckera* sp., *Saccharomycodes* sp., *Schizosaccharomyces* sp., *Yarrowia sp.* or *Zygosaccharomyces* sp. In still enbodiments, the yeast cells are selected from the group consisting of *Saccharomyces* sp., *Brettanomyces* sp., *Candida, Kloeckera* sp., *Saccharomycodes* sp., *Schizosaccharomyces* sp., *Yarrowia sp.* and *Zygosaccharomyces* sp.

In embodiments, the yeast cells can be a *Saccharomyces arboricolus, a Saccharomyces eubayanus, a Saccharomyces bayanus,* a *Saccharomyces beticus, a Saccharomyces cerevisiae,* a *Saccharomyces fermentati, a Saccharomyces kudriadzevii,* a *Saccharomyces mikatae,* a *Saccharomyces paradoxus,* a *Saccharomyces pastorianus* or *Saccharomyces uvarum.* In further embodiments, the yeast cells are selected from the group consisting of *Saccharomyces arboricolus, Saccharomyces eubayanus, Saccharomyces bayanus, Saccharomyces beticus, Saccharomyces cerevisiae, Saccharomyces fermentati, Saccharomyces kudriadzevii, Saccharomyces mikatae, Saccharomyces paradoxus, Saccharomyces pastorianus* and *Saccharomyces uvarum.* In further embodiments, the yeast cells are a *Saccharomyces cerevisiae.*

In embodiments, the yeast cells can be from *Dekkera* sp. In still embodiments, the yeast cells can be from *Pichia* sp., *Metschnikowia* sp., *Issatchenkia* sp., *Torulaspora* sp. or *Kluyveromyces* sp. In yet further embodiments, the yeast cells are selected from the group consisting of *Pichia* sp., *Metschnikowia* sp., *Issatchenkia* sp., *Torulaspora* sp. and *Kluyveromyces* sp. In embodiments, the yeast cells can be from *Hanseniaspora* sp. In further embodiments, the yeast cells can be from *Yarrowia sp.* In still a further embodiment, the yeast cells can be a *Yarrowia lipolytica.*

In embodiments, the at least one polyphenols can be hydrolysable tannins having a molecular weight above about 620 Da. In embodiments, the hydrolysable tannins can have a molecular weight above about 870 Da. In other embodiments, the hydrolysable tannins can have a molecular weight ranging between about 620 Da to about 20 kDa. In further embodiments, the hydrolysable tannins can have a molecular weight ranging between about 870 Da and about 14.5 kDa.

In embodiments, the at least one polyphenols can be proanthocyanidins having a molecular weight above about 620 Da. In embodiments, the proanthocyanidins can have a molecular weight above about 870 Da. In other embodiments, the proanthocyanidins can have a molecular weight ranging between about 620 Da to about 20 kDa. In further embodiments, the proanthocyanidins can have a molecular weight ranging between about 870 Da and about 14.5 kDa.

In embodiments, the at least one polyphenols can be a mixture of proanthocyanidins and hydrolysable tannins having a molecular weight above about 620 Da. In embodiments, the mixture of proanthocyanidins and hydrolysable tannins can have a molecular weight above about 870 Da. In other embodiments, the mixture of proanthocyanidins and hydrolysable tannins can have a molecular weight ranging between about 620 Da to about 20 kDa. In further embodiments, the mixture of proanthocyanidins and hydrolysable tannins can have a molecular weight ranging between about 870 Da and about 14.5 kDa.

In embodiments, the hydrolysable tannins having a molecular weight above 620 Da can have a mean degree of polymerization (DP) ranging between about 3 and about 50. In further embodiments, the hydrolysable tannins having a molecular weight above 620 Da can have a mean degree of polymerization (DP) ranging between about 5 and about 35. In other embodiments, the hydrolysable tannins having a molecular weight above 620 Da can have a mean degree of polymerization (DP) ranging between about 7 and about 25.

In embodiments, the hydrolysable tannins having a molecular weight above 870 Da can have a mean degree of polymerization (DP) ranging between about 3 and about 50. In further embodiments, the hydrolysable tannins having a molecular weight above 870 Da can have a mean degree of polymerization (DP) ranging between about 5 and about 35. In other embodiments, the hydrolysable tannins having a molecular weight above 870 Da can have a mean degree of polymerization (DP) ranging between about 7 and about 25.

In still embodiments, the hydrolysable tannins having a molecular weight ranging between about 620 Da to about 20 kDa can have a mean degree of polymerization (DP) ranging between about 3 and about 50. In further embodiments, the hydrolysable tannins having a molecular weight ranging between about 620 Da to about 20 kDa can have a mean degree of polymerization (DP) ranging between about 5 and about 35. In other embodiments, the hydrolysable tannins having a molecular weight ranging between about 620 Da to about 20 kDa can have a mean degree of polymerization (DP) ranging between about 7 and about 25.

In embodiments, the hydrolysable tannins having a molecular weight ranging between about 870 Da and about 14.5 kDa can have a mean degree of polymerization (DP) ranging between about 3 and about 50. In further embodiments, the hydrolysable tannins having a molecular weight ranging between about 870 Da and about 14.5 kDa can have a mean degree of polymerization (DP) ranging between about 5 and about 35. In other embodiments, the hydrolysable tannins having a molecular weight ranging between about 870 Da and about 14.5 kDa can have a mean degree of polymerization (DP) ranging between about 7 and about 25.

In embodiments, the proanthocyanidins having a molecular weight above 620 Da can have a mean degree of polymerization (DP) ranging between about 3 and about 50. In further embodiments, the proanthocyanidins having a molecular weight above 620 Da can have a mean degree of polymerization (DP) ranging between about 5 and about 35. In other embodiments, the proanthocyanidins having a molecular weight above 620 Da can have a mean degree of polymerization (DP) ranging between about 7 and about 25.

In embodiments, the proanthocyanidins having a molecular weight above 870 Da can have a mean degree of polymerization (DP) ranging between about 3 and about 50. In further embodiments, the proanthocyanidins having a molecular weight above 870 Da can have a mean degree of polymerization (DP) ranging between about 5 and about 35. In other embodiments, the proanthocyanidins having a molecular weight above 870 Da can have a mean degree of polymerization (DP) ranging between about 7 and about 25.

In still embodiments, the proanthocyanidins having a molecular weight ranging between about 620 Da to about 20 kDa can have a mean degree of polymerization (DP) ranging between about 3 and about 50. In further embodiments, the proanthocyanidins having a molecular weight ranging between about 620 Da to about 20 kDa can have a mean degree of polymerization (DP) ranging between about 5 and about 35. In other embodiments, the proanthocyanidins having a molecular weight ranging between about 620 Da to about 20 kDa can have a mean degree of polymerization (DP) ranging between about 7 and about 25.

In embodiments, the proanthocyanidins having a molecular weight ranging between about 870 Da and about 14.5 kDa can have a mean degree of polymerization (DP) ranging between about 3 and about 50. In further embodiments, the proanthocyanidins having a molecular weight ranging between about 870 Da and about 14.5 kDa can have a mean degree of polymerization (DP) ranging between about 5 and about 35. In other embodiments, the proanthocyanidins having a molecular weight ranging between about 870 Da and about 14.5 kDa can have a mean degree of polymerization (DP) ranging between about 7 and about 25.

In embodiments, the mixture of proanthocyanidins and hydrolysable tannins having a molecular weight above 620 Da can have a mean degree of polymerization (DP) ranging between about 3 and about 50. In further embodiments, the mixture of proanthocyanidins and hydrolysable tannins having a molecular weight above 620 Da can have a mean degree of polymerization (DP) ranging between about 5 and about 35. In other embodiments, the mixture of proanthocyanidins and hydrolysable tannins having a molecular weight above 620 Da can have a mean degree of polymerization (DP) ranging between about 7 and about 25.

In embodiments, the mixture of proanthocyanidins and hydrolysable tannins having a molecular weight above 870 Da can have a mean degree of polymerization (DP) ranging between about 3 and about 50. In further embodiments, the mixture of proanthocyanidins and hydrolysable tannins having a molecular weight above 870 Da can have a mean degree of polymerization (DP) ranging between about 5 and about 35. In other embodiments, the mixture of proanthocyanidins and hydrolysable tannins having a molecular weight above 870 Da can have a mean degree of polymerization (DP) ranging between about 7 and about 25.

In still embodiments, the mixture of proanthocyanidins and hydrolysable tannins having a molecular weight ranging between about 620 Da to about 20 kDa can have a mean degree of polymerization (DP) ranging between about 3 and about 50. In further embodiments, the mixture of proanthocyanidins and hydrolysable tannins having a molecular weight ranging between about 620 Da to about 20 kDa can have a mean degree of polymerization (DP) ranging between about 5 and about 35. In other embodiments, the mixture of proanthocyanidins and hydrolysable tannins having a molecular weight ranging between about 620 Da to about 20 kDa can have a mean degree of polymerization (DP) ranging between about 7 and about 25. In embodiments, the mixture of proanthocyanidins and hydrolysable tannins having a molecular weight ranging between about 870 Da and about 14.5 kDa can have a mean degree of polymerization (DP) ranging between about 3 and about 50. In further embodiments, the mixture of proanthocyanidins and hydrolysable tannins having a molecular weight ranging between about 870 Da and about 14.5 kDa can have a mean degree of polymerization (DP) ranging between about 5 and about 35. In other embodiments, the mixture of proanthocyanidins and hydrolysable tannins having a molecular weight ranging between about 870 Da and about 14.5 kDa can have a mean degree of polymerization (DP) ranging between about 7 and about 25. USES

The present disclosure also concerns the use of the composition described herein in cosmetic, dermatological, nutritional and/or pharmaceutical fields.

In embodiments, the composition can be administered orally or topically. Other administration methods known to the skilled person in the art may be used as well. In embodiments, the composition is in the form of a foodstuff, a dietary supplement or feed.

The present disclosure also concerns the use of the composition described herein in oenological field. In embodiments, the composition can be use in winemaking to protect the wine against oxidation, therefore preserving the wine quality, color and aromas. In embodiments, the composition can be used as an alternative to ageing on lees.

In the following examples, we demonstrated that grape and wine polyphenols with molecular weights larger than 620 Da can cross the yeast cell envelope barrier (cell wall and plasma membrane) of dead and/or inactivated yeast and interact with cytoplasm constituents

### EXAMPLE I - Grapes and preparation of skin proanthocyanidins

The grape skin proanthocyanidines fraction was purified from the skin of 1.25 kg Muscat berries. Proanthocyanidines were extracted with 2.5 liters of Acetone/H₂O/Methanol (51/34/15), under stirring and during 2 h.⁸ The mixture was centrifuged for 20 min at 2000 g and 20 °C. The solid phase was extracted again with the same solvent during one night at 4 °C and the mixture was centrifuged. The supernatants were pooled, filtrated, concentrated under vacuum at 30 °C (final volume 200 mL) and stored at - 80 °C before the following steps. Chlorophyll was removed by three extractions with hexane and the proanthocyanidines extract was applied on a styrene/divinylbenzene Diaion resin in batch conditions. The resin was washed several times with H₂O acidified with trifluoroacetic acid (TFA, 0.05% v/v) to remove sugars and proteins (followed by refractometer) and proanthocyanidines were recovered with methanol acidified with Trifluoroacetic acid (0.05 % v/v). Methanol was evaporated and the samples were dispersed in distilled water and freeze-dried. This PA extract was dissolved in H₂O acidified with TFA (0.05% v/v) (5 mL, 47 mg.mL⁻¹) and further applied on a Toyopearl TSK HW-50 (F) gel (Tosoh Corp., Tokyo, Japan) (diameter, 2.7 cm; 18 cm height) equilibrated with the same solvent. The column was then extensively washed with H₂O: TFA (0.05% v/v, 300 mL). Elution by 55:45:0.05 v: v: v EtOH: H2O: TFA (450 mL at 10 mL.min⁻¹) was performed first to remove low molecular weight polyphenols. High molecular weight proanthocyanidins were eluted with a 60:40:0.05 acetone: H2O: TFA v: v: v mixture (450 mL). After concentration under vacuum (rotary evaporator at 30 °C), the high molecular weight PA fraction was freeze-dried and stored at -80 °C. This fraction, named **Skin21,** had a mean degree of polymerization of 20.1 + 0.7 and its percentage of epigallocatechin units was 16.8 + 1.2 %. The yield of the depolymerization reaction was 73.9 %.

### EXAMPLE II - Wine polyphenols

A wine polyphenol pool (WP) was purified from 9 L of a Merlot wine, produced in 2012 at the INRA Experimental Unit of Pech Rouge (Gruissan, France). Separation was achieved by applying the wine to a 7 L bed volume column filled with a styrene/divinylbenzene Diaion resin. The wine flow rate was of 4.2 L.h⁻¹. The resin was rinsed with two bed volumes of distilled water (flow rate 4.2 L.h⁻¹) to remove water soluble compounds. The elution was followed by refractometry. The phenolic pool was finally eluted with 5 L of methanol and 12 L of 96/4 v/v ethanol/water, both solvents being acidified with TFA (0.05%). The ethanolic and methanolic extracts were pooled and the solvents removed by vacuum evaporation at 40 °C. The polyphenol pool was then freeze-dried and stored at - 80 °C under argon atmosphere before further use. The polyphenol composition of the pool was (on a dry weight basis): 29.3 mg.g⁻¹ anthocyanins, 7.7 mg.g⁻¹ flavan-3-ol monomers, 32.7 mg.g⁻¹ phenolic acids, 24.2 mg.g⁻¹ stilbens, 5.9 mg.g⁻¹ flavonols and 95.0 mg.g⁻¹ tannins. The average degree of polymerization of the tannins, determined was 7.3 + 1.1, with a % of epigallocatechin units (EgC) and of epicatechin-gallate (Ec-G) of 24.2 + 2.3 and 3.3 + 0.3, respectively. The low yield was mainly related to the fact that most polyphenols in the wine pool consisted in derived pigments and tannins, formed by chemical reactions during winemaking and aging and resistant to usual depolymerization methods.^{9,10}

### EXAMPLE III - Yeast cells and preparation of yeast cell walls

The yeast strain used in the present work is a commercial *Saccharomyces cerevisiae* strain (Lallemand, Montreal, Canada) commonly used in enology. Yeast was propagated aerobically at 32°C in a fed-batch manner, with a growth rate of 0.18 h⁻¹, for a total of 17 h. Feed substrate was beet molasses diluted to allow suitable delivery of the molasses to the propagation and ammonia hydroxide (5%) was used as the nitrogen source. The yeast biomass (Y) was either frozen at -20 °C and conserved as such or submitted to further post-harvesting treatments to obtain inactivated yeast fractions. The viability of frozen cells (Y), was determined prior interaction assays through flow-cytometry associated with staining techniques and growth on Petri plates. Results (1 and 2.4% respectively) indicated that most cells were dead. A part of the biomass was inactivated by thermal treatment, achieved by holding the yeast cream at 70 °C for 15 minutes in a temperature controlled vessel. The inactivated yeast biomass was spray-dried in a Büchi B290 spray dryer (Büchi, New Castle, DE, USA). This enabled to obtain the inactivated yeast fraction (IY). Another part was submitted to autolysis. Autolysis was carried out by incubating the yeast biomass at a pH of 5.5 at 55°C for 20 h. After autolysis the yeast was centrifuged at 15000 g for 15 min in order to separate the soluble fraction from the insoluble fraction. The insoluble part (A-IY) was spray-dried.

A cell wall fraction (CW) was purified from the biomass Y at the laboratory scale by mechanical disruption, according to the protocol described by Dallies *et al.* (1998).¹¹ Before disruption, the yeast biomass was washed three times in a 5 mM phosphate buffer (pH 7.5) by successive resuspension and centrifugation steps (1000 g, 5 min, 5 °C). Cells were then dispersed in the phosphate buffer at a concentration of 10⁹ cells.mL⁻¹ and 2 mL aliquots distributed in suitable glass tubes containing 5.3 g of 0.5 mm diameter Yttria-Stabilized Zirconium Oxide beads. Complete cell disruption was achieved with a tissue homogenizer (Precellys 24, Bertin Technologies, France) by the application of 13 periods of shaking of 20 s each at 5500 rpm, with 1 min intervals on ice between each period. Cell disruption was followed by optical microscopy. The cell suspension was then collected and beads were extensively washed with the phosphate buffer. The supernatant and washing solution were pooled and centrifuged at 3800 g for 5 min.¹¹ The pellet, containing the cell walls, was washed four times with phosphate buffer and twice with deionized water. The cell wall suspension was heated at 72 °C for 15 min to remove residual enzymatic activities, and sodium azide (0.02% w/v) was added to prevent any microbiological development. The suspension was then stored at 4 °C until use. The dry weight per milliliter of suspension, 4.4 mg.mL⁻¹, was determined by freeze-drying of aliquots. In accordance with literature data,^{1,12} it was calculated that cell walls accounted for about 20% of the whole cell dry weight. Neutral sugars¹¹ and Kjeldhal analyses performed on CW indicated a neutral sugar content of 80% (w/w) and a nitrogen content of about 2% (w/w).

### EXAMPLE IV - Sorption experiments

Suspensions were prepared as described in Chapter 2. Briefly, yeast cells (Y; IY or A-IY) (2*10⁸ cells.mL⁻¹) or yeast cell walls (CW) (0.88 mg.mL⁻¹) were suspended in a model wine (12% ethanol, tartaric acid 2 g.L⁻¹, NaCl 50 mM, pH adjusted to 3.5 with KOH, SO₂ 25 mg.L⁻¹). An equal volume of grape skin tannins or wine polyphenol pool solution (8 mg.mL⁻¹ in model wine) was added to yeast cells or yeast cell wall suspensions, and the mixtures were gently stirred for 24 h. Suspensions were then centrifuged and the pellets recovered. Centrifugation conditions were 1500 g - 5 min - 5°C and 10 000 g - 5 min - 5°C for yeast whole cells and cell walls, respectively. Pellets were washed 4 times with 1 mL of PBS buffer to remove the non sorbed polyphenols, and resuspended in 1 mL of the same buffer. Yeast cells and yeast cell walls suspended in the same conditions in a model wine without polyphenol served as controls.

### EXAMPLE V - Analysis and Results

### Transmission electron microscopy

PBS buffer was removed from the samples and controls and the pellets were re-suspended in a solution of 2% acrolein, 2% glutaraldehyde in cacodylate buffer (0.05M, pH 7) for 12h at 4°C.¹³ They were then rinsed several times in cacodylate buffer and post-fixed in a 1% osmic acid for 2 hours at dark and at room temperature. After two rinses in cacodylate buffer, the samples were dehydrated in a graded series of acetone solutions (30-100%) at room temperature. They were then embedded in EmBed 812 using an Automated Microwave Tissue Processor for Electronic Microscopy, Leica EM AMW. Thin sections (70 nm; Leica-Reichert Ultracut E) were collected at different levels of each block. These sections were stained sequentially with uranyl acetate and lead citrate. They were then observed using a Hitachi 7100 transmission electron microscope with 75 kV accelerating voltage in the Centre de Ressources en Imagerie Cellulaire de Montpellier (France)

### Light and epifluorescence microscopy

Samples and controls were observed under normal light and epifluorescence. Imaging was performed with a Zeiss Axiophot microscope using DAPI filter [DAPI=(4'6-diamidino-2-phenylindole); (λexc = 340-380 nm, λem = 425-800 nm)].

### Confocal microscopy

Confocal imaging was performed with a Zeiss Axiovert microscope 200M 510 META fitted with a Plan-Apochromat x40/1.2 W Zeiss objective. Excitations were obtained for tannins with a 405 nm blue diode, and emissions were collected for tannins with (505-550 nm bandpass) filter.¹⁴ For images acquired in lambda scanning mode, the emission spectra were obtained on sample ROIs (regions of interest) by spectral acquisition (lambda stack, excitation at 405 nm). The detection bandwidth was set to collect emissions from 400 to 750 nm, using an array of 32 photomultiplier tube (PMT) detectors, each with a 10.7 nm bandwidth. The method of linear unmixing was applied with advanced iterative and one residual channel.

### Results

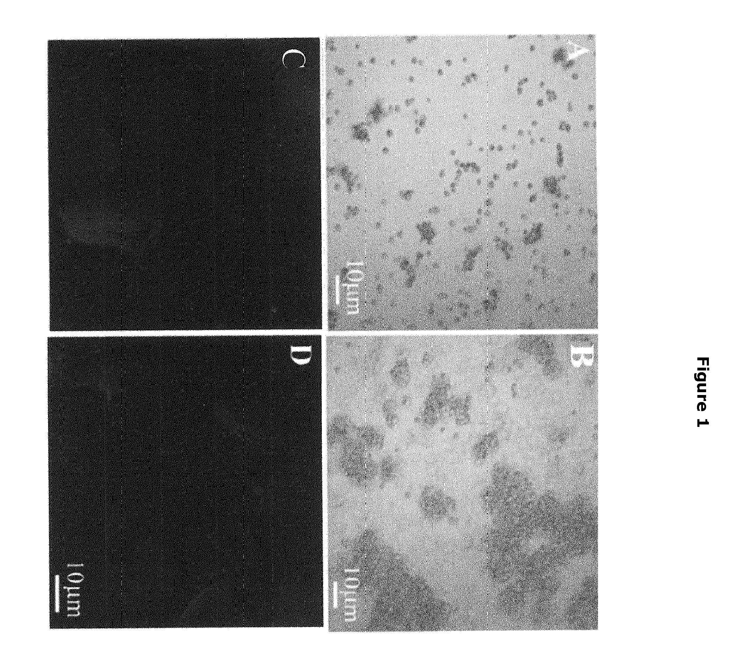

Untreated yeast cells (Y) were observed in light microscopy (Figure. 1A) as regular spheres (average diameter 3 µm), they exhibited no fluorescence (Figure 1C). Yeast cells treated with grape skin proanthocyanidins showed the same morphology than untreated cells, and formed stacks (Figure 1B). They fluoresced strongly in blue (Figure 1D).

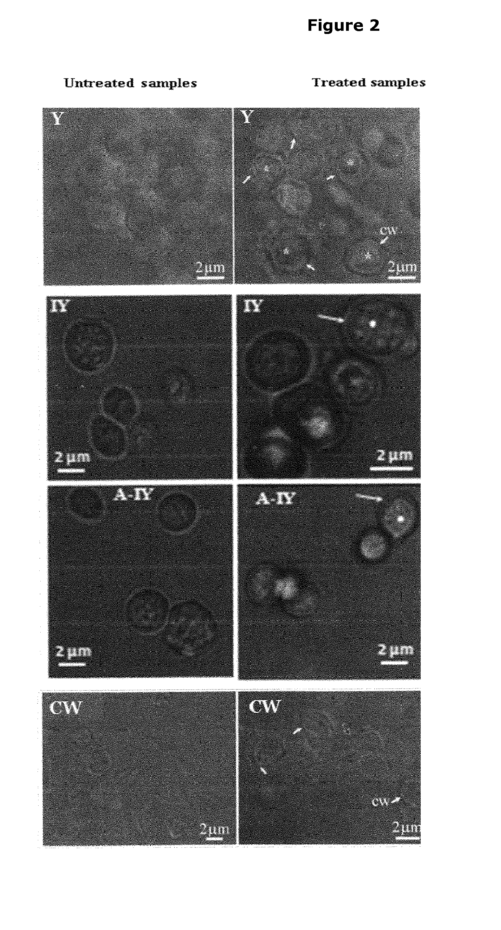

Confocal microscopy was performed on yeast cells (Y), inactivated yeasts (IY, A-IY) and yeast cell wall (CW) (Figure 2). Untreated yeast cells and untreated inactivated yeasts showed an extremely faint and almost invisible blue fluorescence; the same was observed for purified cell walls (Figure 2). After contact with grape skin PAs, yeast cells and inactivated yeasts showed an intense blue fluorescence in their inner core (Figure 2); some plasmolysed cells were observed with Y, they showed in addition to their fluorescent core, a corona fluorescing in blue. Cell walls exhibited a blue fluorescence and were devoid of internal content. In both cases (Y and CW), some scarce residual fluorescent cytoplasmic elements were observed (Figure 2).

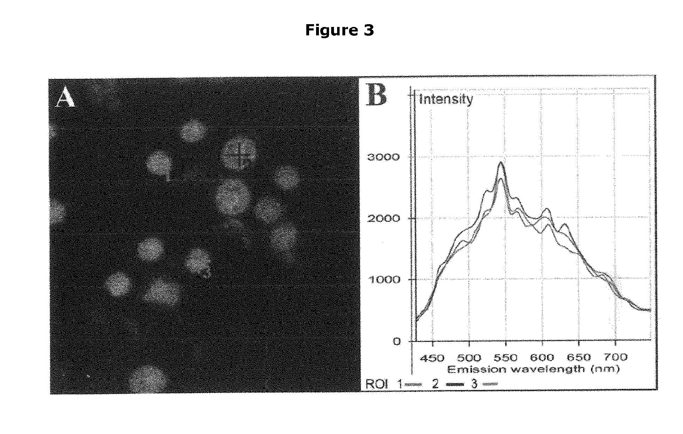

An examination of treated cells (Y) by confocal spectral analysis revealed particles (diameter 3 µm) (Figure. 3A) exhibiting an autofluorescence emission spectrum (Figure. 3B) with several maxima in the 500-600 nm range.

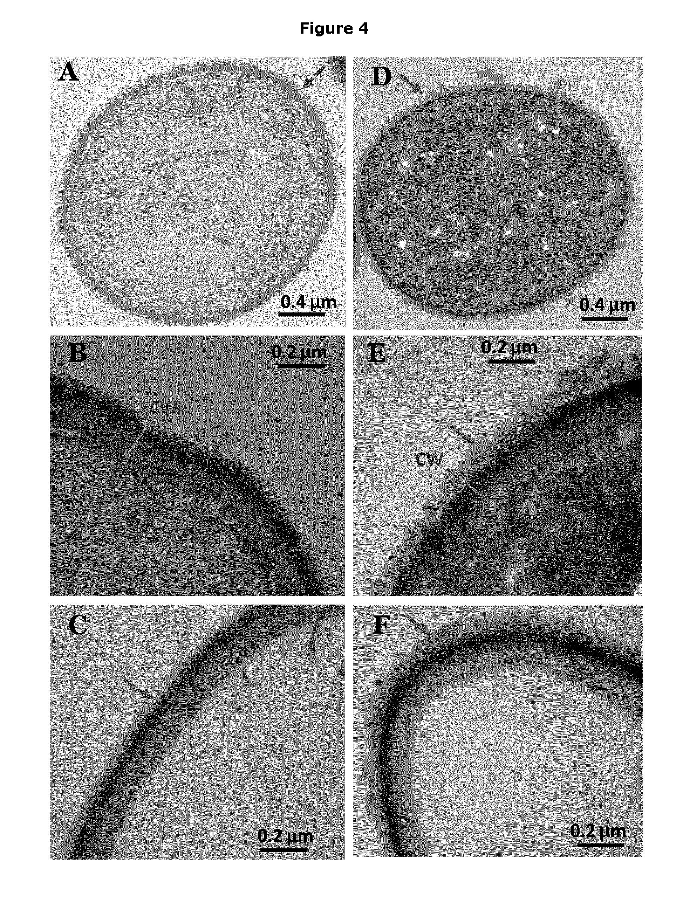

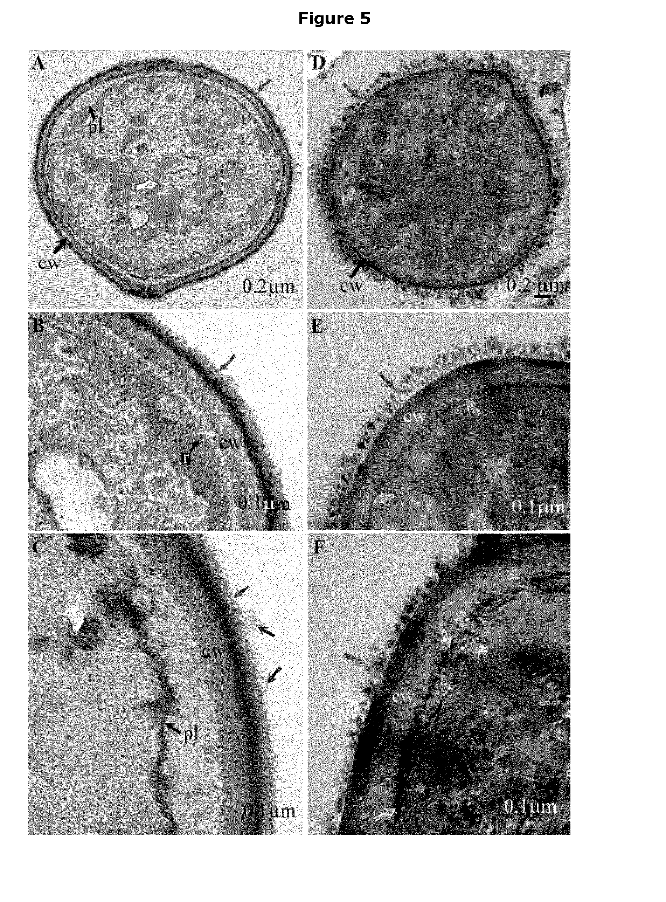

Transmission electron microscopy was performed on yeast cell biomass (Y) and yeast cell wall (CW) before and after treatment with grape skin PAs (Figure 4) or with wine polyphenol pool (Figure 5). Untreated yeast cells (Y) appeared as spherical structure ((Φ= 3 µm) surrounded by a wall (thickness 100-140 nm), itself encircled by a brush-like pattern (thickness 30-40 nm) (Figure. 4A-C; Figure. 5A-C). Cells showed high metabolic activity as shown by myriads of ribosomes in their cytoplasm. Under higher magnification, the outer surface of the wall appeared covered by a vaguely fibrillar network with 4-8 nm contrasted knots (Figure. 4A-C; Figure. 5A-C). Under this brush-like structure, laid a strongly contrasted corona (Figure. 4B, C; Figure. 5B, C), itself deposited on a grey layer.

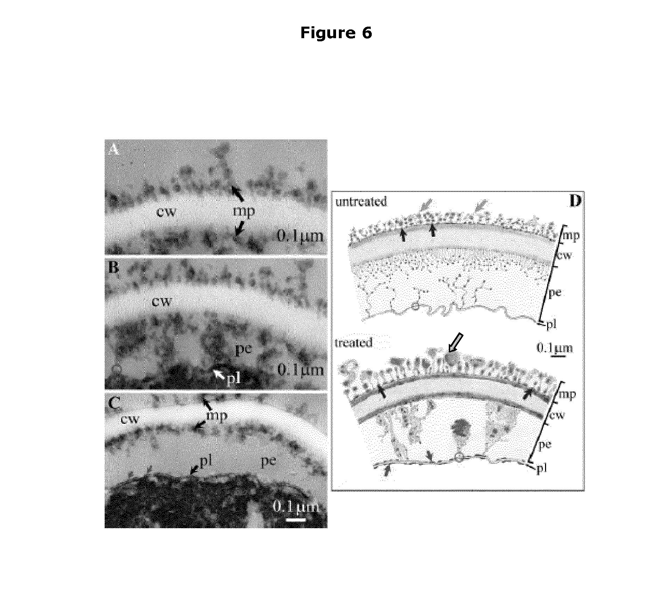

Polyphenol-treated cells and cell walls, compared with untreated cells, showed obvious differences at their cell wall and cytoplasm levels: the outer brush-like pattern appeared as peduculate elements with kind of osmiophilic heads (30-50 nm) (Figure. 4D-F; Figure. 5D-F; Figure. 6A, B). Its thickness migth be as high as the wall *sensu stricto.* Although less dense, similar patterns were observed in plasmolyzed cells on the inner face of the walls (Fig. 6A-C). Finally, at the junction between the cytoplasm and the cell wall, an intensely osmiophilic thin layer was visible (Figure. 5D-F). Another major difference laid in the cytoplasm of polyphenol-treated cells: indeed and contrary to untreated cells, the entire cytoplasm appeared as highly osmiophilic. The plasmalemma (7 nm thickness) appeared in treated cells bearing osmiophilic flat clumps (Figure. 6C).

Yeast treatment with grape skin PAs and wine polyphenols led to the same observations at the yeast cell wall and cytoplasm levels by TEM microscopy. Red wine polyphenols consists in a complex mixture of native polyphenols monomers (mainly phenolic acids, flavanol monomers, anthocyanins) and tannins, along with so-called derived pigments and tannins formed during winemaking due to the chemical reactivity of these constituents.^{9,10,15,16} These structural changes result in the formation of modified units/subunits that cannot be easily analysed and induce bias in the determination of the average degree of polymerization of the latter. In a previous work, we evidenced however that interactions between red wine polyphenols and yeast cells or yeast cell walls mainly concern oligomeric and polymeric forms (unpublished results). Observations similar to those described previously for grape PAs were obtained by microscopy.

### Discussion

The overall morphology of yeast cells - a spherical structure (Φ= 3 µm) bound by a polysaccharide wall (thickness 200 nm), itself covered with a brusk-like pattern, is consistent with previously published images of *Saccharomyces cerevisiae*^{17,18} and *Schizosaccharomyces pombe.* In particular, one must note the regular design of mannoproteins bound to the outer face of the cell wall; it appears as pearling structures extending from the wall with multiple contrasted vaguely spherical elements (thereafter called knots). These structures were decorated with weakly contrasted thin filaments. Plasmolysis of some yeast cells was helpful for description of phenomena: indeed, on intact cells, proteins bound to the outer face of the plasma membrane were hardly visible, plasmalemma being stuck to the wall (Figure. 5A,B); after contact with tannins, only a fine osmophilic layer appressed between wall and plasmalemma was visible (Figure. 5D-F). After plasmolysis, fine structures were unveiled in the space generated: on untreated cells, mannoproteins were seen attached by GP1 anchor (glycosyl-phosphatidylinositol)¹ to the outer face of the plasmalemma (Figure. 6B,D). In this area, we noted that, under the influence of plasmolysis tearing force, the mannoproteins were cleaved, a large proportion remaining attached to the inner face of the wall. The weakly electron-opaque cell wall [mainly β-(1→3)-glucan and chitin] did not show any typical structure.

The unique property of condensed tannins to form insoluble complexes with proteins is well known^{19,20}. This phenomenon is designated in oenology as fining or *collage* (in French); it shows, with regards to the action, a similarity with the clotting of blood, *i.e.* an insolubilization of soluble (plasma) and insoluble (red cells) proteins with shrinking. Yeast cell proteins underwent such an interaction with tannins. Indeed, after contact with grapevine tannins, cells showed a strong blue fluorescence in their cytoplasm; spectral analysis of this fluorescence in comparison with purified grapevine tannins¹⁴ indicated this was due to tannins. Another site of fluorescence, the cell wall, was identified on plasmolysed cells and purified cell walls. Both compartments (cytoplasm and cell wall) contain proteins which therefore engage with tannins into fluorescing complexes.

After contact with the tannins or wine polyphenol pool, the thickness of the brush border doubled in comparison with the untreated cells, and outer mannoproteins became very osmiophilic, indicating presence of polyphenols. Furthermore, the volume and osmiophily of knots increased dramatically, as did the overall volume of the extending structures; fusion of some of them was also visible (Figure. 6D). The inner mannoproteins formed kind of large coagulated osmiophilic structures still attached through their GP1 anchor to the plasmalemma (Figure. 6D); it is possible that the interaction resulted in the fusion of independent mannoproteins, and the osmiophilic knots were still distinguishable, although larger than in untreated cells.

Amongst the multiple effects of condensed tannins, interactions of the plasmalemma proteins with proanthocyanidins were well visible on plasmolysed yeast cells (Figure. 6C). The cytoplasm of treated cells was also much affected by the polyphenols: in comparison with the untreated cells, it appeared highly osmiophilic while untreated cells did not (Figure. 4D-F; Figure. 5D-F); again, it seems that soluble and membrane (ribosomes, endoplasmic reticulum, mitochondrion, nucleus,..) proteins formed complexes with the added exogenous polyphenols. According to their average degree of polymerization, a 3 nm average hydrodynamic volume could be estimated for aDP 21 PAs:²¹ thus, the polysaccharide wall was permeable to this size of molecules.

These results evidenced that interactions between yeast cells and polyphenols are not necessarily limited to cell walls when dealing with dead and/or inactivated cells, and allowed to account for the very large differences observed in our previous work (not published) between whole cells and cell walls towards their ability to interact with grape and red wine tannins.

The proanthocyanidins are liable to enter the periplasmic space through the cell wall and to interact with the cell membrane and its cytoplasmic content. The yeast cells are grown in aerobic condition whereas in traditional winemaking, yeast have performed the alcoholic fermentation and have thus been grown in anaerobic conditions. Indeed, tannin adsorption in the first case was similar to that determined is the second one⁴.

Present results also evidenced that among cell wall polysaccharides (mannoproteins, β-glucans and chitin), mannoproteins are likely primarily involved in the interaction between cell walls and tannins.

While the invention has been described in connection with specific embodiments thereof, it will be understood that the scope of the claims should not be limited by the preferred embodiments set forth in the examples, but should be given the broadest interpretation consistent with the description as a whole.

### References

1. Orlean, P. Architecture and biosynthesis of the Saccharomyces cerevisiae cell wall. Genetics 192, 775-818 (2012).
2. Bonilla, F., Mayen, M., Merida, J. & Medina, M. Yeasts used as fining treatment to correct browning in white wines. J. Agric. Food Chem. 49, 1928-33 (2001).
3. Razmkhab, S., Lopez-Toledano, A., Ortega, J. M., Mayen, M., Merida, J., Medina, M. Adsorption of phenolic compounds and browning products in white wines by yeasts and their cell walls. J. Agric. Food Chem. 50, 7432-7 (2002).
4. Mazauric, J.-P. & Salmon, J.-M. Interactions between yeast lees and wine polyphenols during simulation of wine aging: I. Analysis of remnant polyphenolic compounds in the resulting wines. J. Agric. Food Chem. 53, 5647-5653 (2005).
5. Mazauric, J.-P. & Salmon, J.-M. Interactions between Yeast Lees and Wine Polyphenols during Simulation of Wine Aging: II. Analysis of Desorbed Polyphenol Compounds from Yeast Lees. J. Agric. Food Chem. 54, 3876-3881 (2006).
6. Marquez, T., Millan, C. & Salmon, J.-M. Plasma Membrane Sterols Are Involved in Yeast's Ability To Adsorb Polyphenolic Compounds Resulting from Wine Model Solution Browning. J. Agric. Food Chem. 57, 8026-8032 (2009).
7. Mekoue Nguela, J., Sieczkowski, N., Roi, S. & Vernhet, A. Sorption of Grape Proanthocyanidins and Wine Polyphenols by Yeasts, Inactivated Yeasts and Yeast Cell Walls. J. Agric. Food Chem. (2014). *Submitted.*
8. Mane, C. Phenomenes oxydants et composes phénoliques dans les vins blancs de Champagne: developpements methodologiques pour l'analyse des polymères. PhD thesis. SupAgro Montpellier (2007).
9. Fulcrand, H., Dueñas, M., Salas, E. & Cheynier, V. Phenolic reactions during winemaking and aging. Am. J. Enol. Vitic. 57, 289-297 (2006).
10. Poncet-Legrand, C. Cabane, B., Bautista-Ortin, A. B., Carrillo, S., Fulcrand, H., Perez, J., Vernhet, A. Tannin Oxidation: Intra- versus Intermolecular Reactions. Biomacromolecules 11, 2376-2386 (2010).
11. Dallies, N., François, J.-M. & Paquet, V. A new method for quantitative determination of polysaccharides in the yeast cell wall. Application to the cell wall defective mutants of Saccharomyces cerevisiae. Yeast 14, 1297-306 (1998).
12. Klis, F. M., Boorsma, A. & De Groot, P. W. J. Cell wall construction in Saccharomyces cerevisiae. Yeast 23, 185-202 (2006).
13. Parham, R. A. & Kaustinen, H. M. differentiation of tannin, lipid, and starch in cultured plant cells. inst, pap. chem. appleton, wisconsin (1975).
14. Brillouet, J.-M., Romieu, C., Schoefs, B., Solymosi, K., Cheynier, V., Fulcrand, H., Verdeil, J.-L., & Conéjéro, G. The tannosome is an organelle forming condensed tannins in the chlorophyllous organs of Tracheophyta. Ann. Bot. 112, 1003-14 (2013).
15. McRae, J. M., Falconer, R. J. & Kennedy, J. A. Thermodynamics of grape and wine tannin interaction with polyproline: implications for red wine astringency. J. Agric. Food Chem. 58, 12510-8 (2010).
16. Mouls, L. & Fulcrand, H. UPLC-ESI-MS study of the oxidation markers released from tannin depolymerization: toward a better characterization of the tannin evolution over food and beverage processing. J. Mass Spectrom. 47, 1450-7 (2012).
17. Kapteyn, J. C., Van Den Ende, H. & Klis, F. M. The contribution of cell wall proteins to the organization of the yeast cell wall. Biochim. Biophys 1426, 373-383 (1999).
18. Normand, V., Dardelle, G., Bouquerand, P.-E., Nicolas, L. & Johnston, D. Flavor encapsulation in yeasts: Multitechnique approach for characterization of the release mechanism.. J. Agric. Food Chem. 53, 7532-7543 (2005).
19. Hagerman, A. E. & Butler, L. G. The specificity of proanthocyanidin-protein interactions. J. Biol. Chem. 256, 4494-7 (1981).
20. Charlton, A. J., Baxter, N. J., Khan, L. M., Moir, A.J.G., & Haslam. E., Polyphenol/peptide binding and precipitation. J. Agric. Food Chem. 50, 1593-1601 (2002).
21. Zanchi, D., Konarev, P. V., Tribet, C., Baron, A., Svergun, D. I., & Guyot, S. Rigidity, conformation, and solvation of native and oxidized tannin macromolecules in water-ethanol solution. J. Chem. Phys. 130, (2009).

## Claims

1. A composition comprising yeast cells and at least one polyphenols having a molecular weight above 620 Da, wherein the polyphenols are adsorbed and retained in the cytoplasm of the yeast cell.

2. The composition according to claim 1, wherein the at least polyphenols have a mean degree of polymerization (DP) ranging between about 3 and about 50.

3. The composition according to claim 1 or 2, wherein the at least one polyphenols are selected from the group consisting of flavonols, flavanonols, flavanols, anthocyanidins, [JMN1]anthocyanins, phenolic acids, stilbenoids, hydrolysable tannins and mixtures thereof.

4. The composition according to any one of claims 1 to 3, wherein the at least one polyphenols are formed by polymerization of flavanols.

5. The composition according to anyone of claims 1 to 4, wherein the at least one polyphenols are proanthocyanidins, hydrolysable tannins or mixture thereof.

6. The composition according to anyone of claims 1 to 4, wherein the at least one polyphenols are proanthocyanidins.

7. The composition according to anyone of claims 1 to 4, wherein the at least one polyphenols are hydrolysable tannins.

8. The composition according to anyone of claims 1 to 4, wherein the at least one polyphenols are mixtures of proanthocyanidins and hydrolysable tannins.

9. The composition according to any one of claims 1 to 8, wherein the at least one polyphenols are isolated or extracted from wine, grape skins and seeds, lingonberries and bilberries, cranberries gallnuts, sumac, witch hazel, tea leaves and some types of wood, such as quebracho wood.

10. The composition according to any one on claims 1 to 9, wherein the yeast cells are from *Saccharomyces sp., Brettanomyces sp., Candida, Kloeckera sp., Saccharomycodes sp., Schizosaccharomyces sp., Yarrowia sp. or Zygosaccharomyces sp.*

11. Use of a composition as defined in any one of claims 1 to 10 in cosmetic, dermatological, nutritional and/or pharmaceutical fields.

12. Use of a composition as defined in any one of claims 1 to 10 in winemaking to protect the wine against oxidation, therefore preserving the wine quality, color and aromas.

13. Use of a composition as defined in any one of claims 1 to 10 as an alternative to ageing of wine on lees.
